(19) Europäisches Patentamt
European Patent Office
Office européen des brevets

(11) **EP 2 448 559 B1**

(12) **EUROPÄISCHE PATENTSCHRIFT**

(45) Veröffentlichungstag und Bekanntmachung des Hinweises auf die Patenterteilung:
**11.05.2022   Patentblatt 2022/19**

(21) Anmeldenummer: **10763139.2**

(22) Anmeldetag: **29.06.2010**

(51) Internationale Patentklassifikation (IPC):
*A61K 9/127* (2006.01)     *A61K 9/00* (2006.01)
*A61P 9/12* (2006.01)       *A61P 11/00* (2006.01)

(52) Gemeinsame Patentklassifikation (CPC):
**A61K 9/127; A61K 9/0078; A61P 9/12; A61P 11/00**

(86) Internationale Anmeldenummer:
**PCT/EP2010/059216**

(87) Internationale Veröffentlichungsnummer:
**WO 2011/000835 (06.01.2011 Gazette 2011/01)**

(54) **LIPOSOMEN ZUR PULMONALEN APPLIKATION**

LIPOSOMES FOR PULMONARY APPLICATION

LIPOSOMES DESTINÉS À UNE APPLICATION PULMONAIRE

(84) Benannte Vertragsstaaten:
**AL AT BE BG CH CY CZ DE DK EE ES FI FR GB GR HR HU IE IS IT LI LT LU LV MC MK MT NL NO PL PT RO SE SI SK SM TR**

(30) Priorität: **30.06.2009   DE 102009031274**

(43) Veröffentlichungstag der Anmeldung:
**09.05.2012   Patentblatt 2012/19**

(73) Patentinhaber: **Lung LLC, A Wholly Owned Subsidiary**
**of United Therapeutics Corporation**
**Satellite Beach, FL 32937 (US)**

(72) Erfinder:
• **GESSLER, Tobias**
  **35435 Wettenberg (DE)**

• **SCHMEHL, Thomas**
  **35392 Gießen (DE)**
• **RIEGER, Monika**
  **66582 Spiesen-Elversberg (DE)**

(74) Vertreter: **Patentanwälte Olbricht Buchhold Keulertz**
**Partnerschaft mbB**
**Bettinastraße 53-55**
**60325 Frankfurt am Main (DE)**

(56) Entgegenhaltungen:
WO-A1-02/03959           WO-A1-86/06959
WO-A1-2009/129395        WO-A2-03/084507
WO-A2-2004/112695        WO-A2-2006/124446
US-A1- 2003 232 019      US-A1- 2004 009 231
US-A1- 2006 141 047

**Beschreibung**

Die Erfindung betrifft Liposomen zur pulmonalen Applikation gemäß dem Oberbegriff von Anspruch 1, sowie die Verwendung von Liposomen zur Herstellung von pharmazeutischen Mitteln entsprechend den Ansprüchen 13 bis 16.

Liposomen sind dem Fachmann allgemein bekannt. Dabei handelt es sich um kolloidale Partikel, die sich spontan bei der Vermischung von Phospholipiden mit einem wässrigen Medium bilden. Ein für die medizinische Anwendung solcher Liposomen besonders vorteilhaftes Merkmal ist es, dass sich die Phospholipide bei der Bildung der Liposomen in Form einer Membran organisieren, die der natürlichen Membran von Zellen und Zellorganellen sehr ähnlich ist. Gleichzeitig wird im Inneren der Liposomen ein gewisser Teil der wässrigen Lösung eingekapselt. Liposomen können daher sowohl als Träger für fettlösliche - in der Membran gelagerte - als auch als Träger für wasserlösliche - in der eingekapselten wässrigen Lösung gelagerte - therapeutische Wirkstoffe eingesetzt werden.

Zur Verabreichung von geträgerten und ungeträgerten Wirkstoffen sind eine Reihe von Möglichkeiten bekannt. So ist es allgemein üblich, pharmazeutische Formulierungen oral beispielsweise in Form von Tabletten oder Flüssigkeiten zuzuführen. Dies hat den großen Nachteil, dass Träger und/oder Wirkstoffe - sofern nicht unmittelbar für den Magen bestimmt - zunächst die aggressive Magenumgebung überstehen müssen, bevor sie über den Darm absorbiert werden können und in den Blutkreislauf gelangen. Als nächstes müssen sie dann noch durch den Körper zum unmittelbaren Bestimmungsort transportiert werden. Eine präzise und zielgerichtete Wirkstoffzufuhr zu einem erkrankten Organ bzw. spezifischen Gewebe ist damit nur begrenzt möglich. Stattdessen werden darüber hinaus auch gesunde Organe und Gewebe mit Wirkstoff versorgt, für welche dieser möglicherweise gerade schädlich ist und es treten daher häufig unerwünschte Nebenwirkungen auf. Gleichzeitig wird die Menge des tatsächlich am Zielort ankommenden Wirkstoffes dadurch drastisch verringert. Mithin ist es häufig notwendig eine erheblich größere Menge oft teuren Wirkstoffs zu verabreichen, als eigentlich zur Therapie benötigt wird.

Um dies zu umgehen, ist man häufig bemüht, eine Möglichkeit zur Verabreichung unter Umgehung des Verdauungstraktes direkt am Wirkungsort oder in dessen unmittelbarer Umgebung zu finden. Neben beispielsweise der intravenösen, intraperitonealen oder intramuskulären Verabreichung hat sich dabei insbesondere die Inhalation von Wirkstoffen als für den Patienten vorteilhaft und angenehm herausgestellt. Sie eignet sich beispielsweise zur Behandlung von systemischen Erkrankungen wie z.B. Diabetes mellitus und ist vorteilhaft bei der Behandlung von Erkrankungen des Respirationstraktes, beispielsweise pulmonaler Hypertonie (vgl. Kleemann et al., Pharmaceutical Research, Vol 24, No.2, Februar 2007) aber auch COPD, Asthma und Lungenentzündungen. Ein wesentlicher Nachteil der herkömmlichen Aerosoltherapien ist die meist kurze Wirkdauer der inhalativ verabreichten Wirkstoffe. Es ist in der Folge meist nötig, Inhalationen in kurzen Abständen durchzuführen. Beispielsweise sind für die Behandlung der pulmonalen Hypertonie mit inhalativem Iloprost bis zu 12 Inhalationen pro Tag mit einer Dauer von jeweils etwa 10 Minuten erforderlich Dies schränkt die Lebensqualität der Patienten jedoch erheblich ein. Außerdem treten relativ hohe Wirkstoffkonzentrationen unmittelbar während oder nach der inhalativen Applikation auf, wohingegen in den Inhalationspausen quasi kein Wirkstoff zur Verfügung gestellt wird. Dies birgt unter anderem die Gefahr, dass es in der Nacht, wenn keine Inhalationen durchgeführt werden, rasch zu einer Wirkstoff-Unterversorgung des Patienten kommen kann.

Eine Grundvoraussetzung für eine effektive Inhalationstherapie ist die Lungengängigkeit der eingesetzten Aerosolpartikel. Diese hängt insbesondere vom Durchmesser und der Dichte der Partikel ab.

Ein kritischer Punkt für die inhalative Verabreichung von Liposomen ist außerdem deren Stabilität gegenüber dem Prozess der Verneblung. Während der Verneblung von Suspensionen und Flüssigkeiten treten verschiedene Kräfte auf, die die Integrität von darin enthaltenen Liposomen beinträchtigen können. In der Folge kann es zu einer Freisetzung des in den Liposomen verkapselten Wirkstoffes kommen.

Die DE 102 14 983 A sieht daher vernebelbare Liposomen zur pulmonalen Applikation von Wirkstoffen vor. Hauptbestandteil der dort offenbarten liposomalen Formulierungen ist Dipalmitoylphosphatidylcholin (DPPC), welches im Verhältnis von 7:3 bzw. 7:4 mit Cholesterin (Chol) gemischt wird. Zusätzlich wird als dritte Komponente Dimyristoylphosphatidylcholin (DMPC), Polyethylenglykol (PEG) oder Sphingomyelin (SM) zugesetzt. Diese Liposomen werden vernebelt und können so vom Patienten inhaliert werden. Nachteilig bei diesen Formulierungen ist jedoch insbesondere die begrenzte Stabilität der Liposomen gegenüber der Verneblung. In der Folge erreicht bei einer Vernebelung der Liposomen zur Inhalation nur ein Bruchteil der wirkstoffbeladenen Liposomen tatsächlich intakt die Lunge. Außerdem zeigen diese Liposomen nur einen begrenzten Controlled Release-Effekt in der Lunge.

Aus US 2003/232019 A1 ist eine Formulierung zur pulmonalen Anwendung bekannt, enthaltend einen aktiven Wirkstoff, ein Phospholipid sowie optional Cholesterin, DSPC oder DPPC.

WO 86/06959 A1 zeigt verschiedene Liposomenzusammensetzungen, die Disteaorylphosphatidylcholin aufweisen.

Aus WO 2004/112695 A2 und US 2006/141047 A1 sind unter anderem Liposomen bestehend aus Disteaorylphophatidylcholin (DSPC), Cholesterin und PEG-DSPE in einem molaren Verhältnis von 9:5:1 bekannt.

Schließlich offenbart WO 03/084507 A2 Liposomen enthaltend Dipalmitoylphosphatidylcholin (DPPC), Cholesterin und Dimyristoylphosphatidylcholin (DMPC), Polyethylenglykol oder Sphingomyelin.

Wünschenswert wäre stattdessen eine retardierte Freisetzung des Wirkstoffes aus einer pulmonal verabreichten liposomalen Formulierung über einen längeren Zeitraum mit dem Ziel einer kontinuierlichen Wirkstoffversorgung.

Aufgabe der Erfindung ist es daher, diese und andere Nachteile des Standes der Technik zu überwinden und Liposomen zur Verfügung zu stellen, die eine hohe Stabilität gegenüber Vernebelung aufweisen. Gleichzeitig sollen die aus liposomalen Formulierungen hergestellten Aerosole gut lungengängig und die Liposomen biologisch gut verträglich sein, sowie eine verzögerte Freisetzung von eingeschlossenen Wirk- und/oder Farbstoffen im Zielgewebe ermöglichen. Die Liposomen sollen darüber hinaus möglichst einfach, zuverlässig und kostengünstig herstellbar sein. Weiterhin soll die Möglichkeit geschaffen werden, pharmazeutische Mittel herzustellen, die zur Prävention, Diagnose und/oder Behandlung von systemischen Erkrankungen und von Lungenerkrankungen geeignet sind.

Hauptmerkmale der Erfindung sind in Anspruch 1 sowie in den Ansprüchen 13 bis 16 angegeben. Ausgestaltungen sind Gegenstand der Ansprüche 2 bis 12.

Zur Lösung der Aufgabe sieht die Erfindung eine Liposomale Formulierung für die pulmonale Applikation mittels Vernebelung vor, wobei die Formulierung Folgendes enthält:

    A) Liposomen umfassend

        i) ein erstes Phospholipid, das Disteaorylphosphatidylcholin (DSPC) ist,
        ii) ein zweites Phospholipid, das ein Phosphatidylcholin oder ein Ethanolamin ist, ausgewählt aus der Gruppe bestehend aus Dimyristoylphosphatidylcholin (DMPC), Dipalmitoylphosphatidylcholin (DPPC) und Dipalmitoylphosphatidylethanolamin (DPPE), und
        iii) Cholesterin und

    B) mindestens einen in Liposomen eingeschlossenen Wirkstoff
    und/oder Farbstoff

Solche erfindungsgemäßen Liposomen können mit hoher Effizienz Wirkstoffe an einen Zielort wie zum Beispiel die Lunge transportieren, ohne dass die Integrität der Liposomen auf dem Transportweg wesentlich beeinträchtigt wird. Außerdem werden eingekapselte Wirkstoffe beim Erreichen des Zielortes nicht schlagartig auf einmal, sondern über einen langen Zeitraum abgegeben. Die erfindungsgemäßen Liposomen sind daher besonders gut geeignet für einen Einsatz, bei dem der enthaltene Wirkstoff retardiert freigesetzt werden soll, beispielsweise einer Freigabe entsprechend dem Sustained release-Typ. Dem Patienten bleiben dabei vielfache und lange andauernde Inhalationen erspart. Stattdessen nimmt er mit einer Inhalation die komplette für einen längeren Zeitraum benötigte Menge an Wirkstoff auf. Dieser wird jedoch durch die Liposomen zunächst zurückgehalten und im Zielgewebe kontinuierlich in solchen Dosen abgegeben, dass die erwünschte therapeutische Wirkung eintreten kann, negative Effekte durch Überdosierung jedoch vermieden werden. Mithin wird eine kontinuierliche Versorgung mit Wirkstoff auf einem konstanten Level gewährleistet. Die Lebensqualität der Patienten wird aufgrund der geringeren Anzahl notwendiger Inhalationen positiv beeinflusst.

All dies wird besonders begünstigt, wenn das zweite Phospholipid DMPC oder DPPE, besonders bevorzugt DPPE ist und wenn das erste und das zweite Phospholipid in einem molaren Verhältnis von 0,5:1 bis 10:1, bevorzugt in einem Verhältnis von 6:1 bis 2:1, besonders bevorzugt in einem Verhältnis von 3:1 vorliegen. Außerdem ist es vorteilhaft, wenn das molare Verhältnis zwischen Phospholipiden und Cholesterin zwischen 10:1 und 1:1, bevorzugt zwischen 6:1 und 3:1, besonders bevorzugt 4:1 beträgt.

Außerdem ist es von besonderem Vorteil, wenn die Liposomen eine Vernebelungsstabilität von mehr als 50%, bevorzugt mehr als 75%, besonders bevorzugt mehr als 80% aufweisen. Eine solche hohe Stabilität der Liposomen verhindert unter anderem effizient, dass bei der Vernebelung durch Zerstörung der Liposomen unverpackter Wirkstoff in das Inhalat freigesetzt wird. In der Folge wird verhindert, dass durch unverkapselt vorliegenden Wirkstoff im Inhalat eine Überdosierung erfolgt oder unerwünschte Nebenwirkungen auftreten.

Weiterhin ist es von Vorteil, wenn der mediane Durchmesser der Liposomen zwischen 0,05 $\mu$m und 5 $\mu$m, bevorzugt zwischen 0,2 $\mu$m und 2,0 $\mu$m beträgt. Mithin sind die Liposomen kleiner als die bei der Vernebelung gebildeten Aerosolpartikel. Bei diesen handelt es sich um kleine Tropfen, die jeweils eine Vielzahl der erfindungsgemäßen Liposomen enthalten. Günstig ist es auch, wenn der mediane aerodynamische Volumen-Durchmesser von Aerosolpartikeln, in denen die Liposomen enthalten sind, zwischen 1 $\mu$m und 6 $\mu$m, bevorzugt zwischen 1,5 $\mu$m und 5 $\mu$m, besonders bevorzugt zwischen 2 $\mu$m und 4,5 $\mu$m beträgt. Man erkennt mit Blick auf die Vernebelungsstabilität, dass es vorteilhaft ist, wenn sich die Größe der Liposomen nach der Vernebelung um weniger als 1 $\mu$m, bevorzugt weniger als 0,2 $\mu$m von der Größe der Liposomen vor der Vernebelung unterscheidet.

Insbesondere in Bezug auf die retardierte Wirkstofffreisetzung ist es außerdem besonders günstig, wenn die Phasenübergangstemperatur der Liposomen höher als 37°C, bevorzugt höher als 45°C, besonders bevorzugt höher als 50°C ist. So liegen die Phospholipide der Liposomen unterhalb dieser Phasenübergangstemperatur in einem quasikristallinen Gitterwerk vergleichsweise starr und unbeweglich vor. Eingeschlossene Wirkstoffe können die Lipidmembran dieser Liposomen nur schwer durchdringen und werden daher nur langsam und in geringem Maße freigesetzt. Oberhalb der Phasenübergangstemperatur befinden sich die Phospholipide in einem flüssigkristallinen Zustand, was eine schnellere Diffusion eingeschlossener Stoffe durch die Membran der Liposomen erlaubt. Wirkstoffe werden dann sehr schnell freigesetzt. Ist nun eine verzögerte Wirkstofffreisetzung gewünscht ist, sieht man leicht ein, dass es günstig ist, wenn die Phasenübergangstemperatur der erfindungsgemäßen Liposomen oberhalb der Körpertemperatur, d. h. oberhalb von 37°C, liegt.

Man erkennt weiterhin, dass die erfindungsgemäßen Liposomen vorteilhaft mit piezoelektrischen, Düsen-, Ultraschall-Aerosolgeneratoren oder Soft-Mist-Inhalern vernebelbar sind. Darüber hinaus können sie zur Herstellung eines pharmazeutischen Mittels zur Prävention, Diagnose und/oder Behandlung von Lungenerkrankungen und/oder systemischen Erkrankungen verwendet werden.

Als Wirkstoffe können mit Vorteil solche eingesetzt werden, die ausgewählt sind aus der Gruppe Appetitzügler/Antiadiposita, Azidosetherapeutika, Analeptika/Antihypoxämika, Analgetika, Antirheumatika, Anthelminthika, Antiallergika, Antianämika, Antiarrhythmika, Antibiotika, Antiinfektiva, Antidementiva, Antidiabetika, Antidota, Antiemetika, Antivertiginosa, Antiepileptika, Antihämorrhagika, Hämostatika, Antihypertonika, Antihypoglykämika, Antihypotonika, Antikoagulantia, Antiomykotika, Antiparasitika, Antiphlogistika, Antitussiva, Expektorantia, Arteriosklerosemittel, ß-Blocker, Kalzium-Kanal-Blocker, Hemmstoffe des Renin-Angiotensin-Aldosteron-Systems, Broncholytika, Antiasthmatika, Cholagoga, Gallenwegstherapeutika, Cholinergika, Kortikoide, Diagnostika und Mittel zur Diagnosevorbereitung, Diuretika, Durchblutungsfördernde Mittel, Entwöhnungsmittel, Enzyminhibitoren, Enzymaktivatoren oder -stimulatoren, Präparate bei Enzymmangel, Rezeptorantagonisten, Transportproteine, Fibrinolytika, Geriatrika, Gichtmittel, Grippemittel, Mittel gegen Erkältungskrankheiten, Gynäkologika, Hepatika, Hypnotika, Sedativa, Hypophysen- oder Hypothalamushormone, regulatorische Peptide, Hormone, Peptid-Hemmstoffe, Immunmodulatoren, Kardiaka, Koronarmittel, Laxantia, Lipidsenker, Lokalanästhetika, Neuraltherapeutika, Magen-Darm-Mittel, Migränemittel, Mineralstoffpräparate, Muskelrelaxanzien, Narkosemittel, Neurotrope Mittel, Osteoporosemittel, Kalzium-/ Knochenstoffwechsel-Regulatoren, Parkinsonmittel, Psychopharmaka, Sinusitismittel, Roborantia, Schilddrüsentherapeutika, Sera, Immunglobuline, Impfstoffe, Antikörper, Sexualhormone und ihre Hemmstoffe, Spasmolytika, Anticholinergika, Thrombozytenaggregationshemmer, Tuberkulosemittel, Urologika, Venentherapeutika, Vitamine, Zytostatika, Antineoplatische Mittel, Homöopathika, Gefäßaktive Substanzen, Gentherapeutika (DNA/RNA-Derivate), Transkriptions-Inhibitoren, Virostatika, Nikotin, Mittel gegen erektile Dysfunktion, Stickstoffmonoxid und/oder Stickstoffmonoxid-liberierende Substanzen.

Auch magnetische Partikel können Wirk- und/oder Farbstoffe im Sinne der vorliegenden Erfindung sein. Solche Partikel können beispielweise in diagnostischen bildgebenden Verfahren, aber auch in der Therapie, z.B. in der Chemo- und Strahlentherapie und der Hyperthermie, zum Einsatz kommen.

Bei den Diagnostika kann es sich sowohl um *in vitro*- als auch um *in vivo*-Diagnostika handeln. Ein erfindungsgemäß einzusetzendes Diagnostikum kann beispielsweise bildgebend und/oder radioaktiv und/oder ein Kontrastmittel sein.

Insbesondere ist die Verwendung der Liposomen zur Herstellung eines pharmazeutischen Mittels zur Prävention, Diagnose und/oder Behandlung von Erkrankungen des Alveolarraumes sowie zur Behandlung von Erkrankungen der Atemwege und die Verwendung der Liposomen zur Herstellung eines pharmazeutischen Mittels zur Prävention, Diagnose und/oder Behandlung der pulmonalen Hypertonie von besonderem Vorteil.

So können die erfindungsgemäßen Liposomen zur Herstellung von Mitteln zur Behandlung oder Diagnose folgender Erkrankungen eingesetzt werden: Entzündliche (infektiöse, nicht-infektiöse) und hyperproliferative (neoplastische, nicht-neoplastische) Erkrankungen der Lunge und der Atemwege, wie Bronchitis, COPD, Asthma, Pneumonie, Tuberkulose, pulmonale Hypertonie, Lungentumoren, fibrosierende Lungenerkrankungen. Weiterhin Lungenmetastasen, Mukoviszidose, Sarkoidose, Aspergillose, Bronchiekatsien, ALI, IRDS, ARDS, Alveolarproteinose, Immunsuppression und Infektionsprophylaxe nach Lungentransplantation.

Auch der Einsatz bei Sepsis, Fettstoffwechselstörungen, Tumorerkrankungen, Leukämien, angeborenen Stoffwechselstörungen (z. B. Wachstumsstörungen, Speicherstörungen, Störungen des Eisenhaushalts), endokrinologischen Erkrankungen, beispielsweise der Hypophyse oder der Schilddrüse (Glandula thyreoidea), Diabetes, Adipositas, psychischen Erkrankungen (z.B. Schizophrenie, Depression, bipolar-affektive Störungen, posttraumatisches Stresssyndrom, Angst- und Panikstörungen), ZNS-Erkrankungen (beispielsweise M. Parkinson, Multiple Skerose, Epilepsie), Infektionskrankheiten, rheumatischen Erkrankungen, allergischen und Autoimmunerkrankungen, erektilen Dysfunktionen, Erkrankungen des Herz-Kreislaufsystems (beispielsweise Arterielle Hypertonie, Koronare Herzerkrankung, Herzrhythmusstörungen, Herzinsuffizienz, Thrombosen und Embolien), Niereninsuffizienz, Anämien, Antikörpermangelsyndromen, angeborenen oder erworbenen Gerinnungsstörungen, Thrombzytenfunktionsstörungen oder Vitaminmangelsyndromen ist denkbar.

Weitere Merkmale, Einzelheiten und Vorteile der Erfindung ergeben sich aus dem Wortlaut der Ansprüche sowie aus der folgenden Beschreibung von Ausführungsbeispielen und anhand der Figuren. Es zeigen:

Fig. 1 die liposomalen Formulierungen der Ausführungsbeispiele 1, 2 und 3,

Fig. 2 die Wirkstoff-Beladungs-Effizienz und Liposomenstabilität nach Vernebelung,

Fig. 3 die Parameter der Aerosol-Partikelgrößenverteilung (MMAD, GSD) verschiedener Liposomendispersionen,

Fig. 4a die Freisetzungscharakteristik verschiedener Wirkstoff-beladener Liposomen in PBS,

Fig. 4b die Freisetzungscharakteristik verschiedener Wirkstoff-beladener Liposomen in PBS/Surfactant,

Fig. 5 die Freisetzungscharakteristik verschiedener Wirkstoff-beladener Liposomen im isolierten Organmodell

Um die Merkmale der erfindungsgemäßen Liposomen genauer zu charakterisieren werden drei erfindungsgemäße Ausführungsbeispiele für liposomale Formulierungen in Bezug auf Wirkstoff-Beladungseffizienz, Wirkstoff-Einschluss nach Vernebelung (Stabilität), Phasenübergangstemperatur, Aerosolpartikel- und Liposomengröße sowie Wirkstoff-Freisetzung näher beschrieben.

In allen drei Beispielen wird als Modell-Wirkstoff der wasserlösliche Fluoreszenzfarbstoff Carboxyfluorescein CF eingesetzt. Je nach der gewünschten Anwendung der Liposomen ist jedoch ein beliebiger anderer wasser- oder fettlöslicher oder amphiphiler Wirkstoff denkbar, bspw. Iloprost, Sildenafil, Treprostinil, blutdrucksenkende Mittel, Insulin, verschiedene Antibiotika etc. oder auch ein Vitalfarbstoff, Kontrastmittel oder ein beliebiger anderer Marker. So können die Liposomen beispielsweise auch im Bereich der Diagnostik eingesetzt werden.

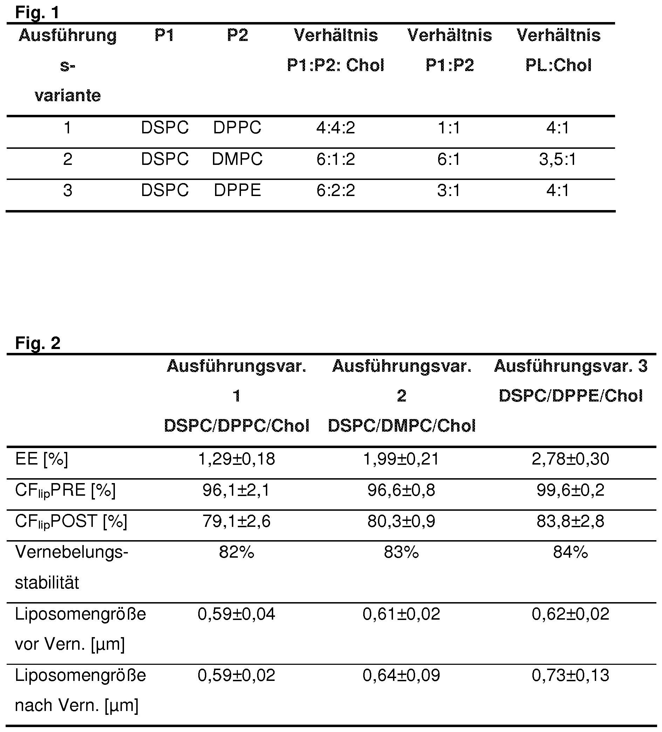

Die Liposomen werden jeweils entsprechend der an sich bekannten Filmmethode wie folgt präpariert: Eine erfindungsgemäße Lipidmischung von erstem und zweitem Phospholipid P1, P2, PL mit Cholesterin Chol (im Ganzen 150 mg, für die Mischungsverhältnisse siehe Fig. 1 und Ausführungsbeispiele 1 bis 3) wird in 40 ml einer Lösungsmittel-Mischung aus sieben Teilen Chloroform und drei Teilen Methanol aufgelöst.

Anschließend wird das Lösungsmittel-Gemisch mit Hilfe eines Rotationsverdampfers (beispielsweise Rotavapor M. Büchi Labortechnik, Flawil, CH) für eine Stunde unter Unterdruck und oberhalb der Phasenübergangstemperatur der Lipidmischung entfernt. Dadurch wird ein dünner Lipidfilm erhalten, der dann für eine weitere Stunde im Vakuum getrocknet wird.

Der Modell-Wirkstoff Carboxyfluorescein CF wird in PBS-Puffer mit pH 7.4 in einer Konzentration von 50 mg/ml gelöst. Diese Lösung wird auf 65°C erhitzt.

Nach dem Trockenen des Lipidfilms werden 10 ml der erhitzten Carboxyfluoresceinenthaltenden Pufferlösung zu dem Lipidfilm gegeben. Um eine Einkapselung des Modell-Wirkstoffes zu bewirken, wird der den so rehydrierten Film enthaltende Kolben für zwei Stunden bei 65°C rotiert. Um die Bilayer-Membranen in der entstandenen Dispersion von multilamellaren Liposomen zu stabilisieren, wird die Dispersion im Anschluss für eine Stunde bei 4°C gelagert.

Dann wird die Größe der in der so hergestellten Dispersion enthaltenen Vesikel reduziert. Dazu wird die Dispersion 21 mal bei 70°C mit Hilfe eines Hand-Extruders (beispielsweise Liposofast, Avestin, Ottawa, Canada) durch eine 400 nm Polycarbonatmembran (beispielsweise von Avestin, Mannheim, Deutschland) extrudiert. Die so erhaltenen Liposomen werden wiederum zur Stabilisation für 20 Stunden bei 4°C gelagert.

Um die Carboxyfluorescein-beladenen Liposomen von in der Lösung enthaltenem nichteingekapseltem, freiem Carboxyfluorescein CF zu trennen, wird die Dispersion viermal bei 4°C, 4500 RZB und 210 r/mm zentrifugiert. Der Überstand, der das freie Carboxyfluorescein CF enthält, wird jeweils abgenommen und die Liposomen-Pellets in einem dem Überstand entsprechenden Volumen PBS-Puffer resuspendiert.

Zur Bestimmung der in Fig. 2 dargestellten Wirkstoff-Beladungs-Effizienz EE verschiedener erfindungsgemäßer liposomaler Formulierungen (vgl. Fig. 1 und Ausführungsbeispiele 1 bis 3 für die Zusammensetzung der Formulierungen) wird sowohl die Konzentration nicht-eingekapselten Carboxyfluoresceins $C_{free}$ als auch die Gesamtkonzentration Carboxyfluorescein $C_{tot}$ fluorimetrisch gemessen. Für die Messung der Konzentration nicht-eingekapselten Carboxyfluoresceins $C_{free}$ werden beispielsweise 100 µl der Liposomen-Dispersion abzentrifugiert und die Konzentration von Carboxyfluorescein CF im Überstand bestimmt. Für die Messung der Gesamtkonzentration $C_{tot}$ werden 100 µl der zu messenden Lösung mit 900 µl 1% Triton-X 100/PBS-Lösung aufgefüllt und für 10 Minuten bei Raumtemperatur geschüttelt, so dass das eingekapselte Carboxyfluorescein CF freigesetzt wird.

Die Wirkstoff-Beladungs-Effizienz EE der liposomalen Formulierungen errechnet sich dann aus folgender Gleichung und wird in Prozent [%] angegeben

$$EE[\%] = 100 * C_{encaps}/C_{start}$$

Dabei beschreibt $C_{encaps}$ die Konzentration des eingekapselten Wirkstoffs und $C_{start}$ die bei der Herstellung eingesetzte Wirkstoffkonzentration (hier 50mg/ml). Die Konzentration des eingekapselten Wirkstoffs $C_{encaps}$ errechnet sich gemäß folgender Formel:

$$C_{encaps} = C_{tot} - C_{free}$$

Man erkennt, dass die beispielhaft gezeigten erfindungsgemäßen Liposomen eine in Bezug auf hydrophile Wirkstoffe für die Filmmethode typische Wirkstoff-Beladungs-Effizienz EE von etwa 1 bis 3 % aufweisen.

Die ebenfalls in Fig. 2 dargestellte Stabilität der Liposomen nach der Vernebelung wird wie folgt bestimmt. Zur Vernebelung mit Hilfe eines piezoelektrischen Vernebelers (Aeroneb Pro®, Aerogen, Irland) werden die oben beschriebenen Liposomen-Suspensionen mit PBS-Puffer derart verdünnt, dass der Gehalt an Carboxyfluorescein CF 500 $\mu$g/ml beträgt. 3 ml der so erhaltenen Lösung werden vernebelt. Aus dem Nebel werden Proben gesammelt, indem eine Glasplatte in das Aerosol eingebracht und der sich dort sammelnde Niederschlag aufgefangen wird. Für jede dieser Proben wird sowohl der freie Carboxyfluorescein-Gehalt $C_{free}$ als auch die Gesamt-Konzentration an Carboxyfluorescein $C_{tot}$ wie oben beschrieben bestimmt. Daraus berechnet sich der prozentuale Anteil eingekapselten Carboxyfluoresceins $CF_{lip}$ aus der Gleichung

$$CF_{lip} = 100\% * (C_{tot} - C_{free})/C_{tot}$$

Die Stabilität der Liposomen gegenüber der Vernebelung lässt sich dann durch den Vergleich des vor der Vernebelung bestimmten Anteils eingekapselten Carboxyfluoresceins $CF_{lip}$pre mit dem nach der Vernebelung bestimmten Anteil eingekapselten Carboxyfluoresceins $CF_{lip}$post erkennen.

Dabei kann der prozentuale Anteil stabiler Liposomen, also die Vernebelungsstabilität, durch das Verhältnis 100*($CF_{lip}$post):($CF_{lip}$pre) beschrieben werden. Man erkennt, dass die beispielhaft gezeigten erfindungsgemäßen Liposomen eine Vernebelungsstabilität von mehr als 80 % aufweisen.

Weiterhin zeigt Fig. 2, dass auch die Größe der erfindungsgemäßen Liposomen eine hohe Stabilität gegenüber der Vernebelung aufweist. Um dies zu bestimmen, werden 100 bis 200 $\mu$l der Liposomen-Dispersion vor und nach der Vernebelung in 40 ml aqua dest. verdünnt und der Mediane Volumen-Durchmesser MVD der Liposomen mit Hilfe von Laser-Lichtstreuung gemessen.

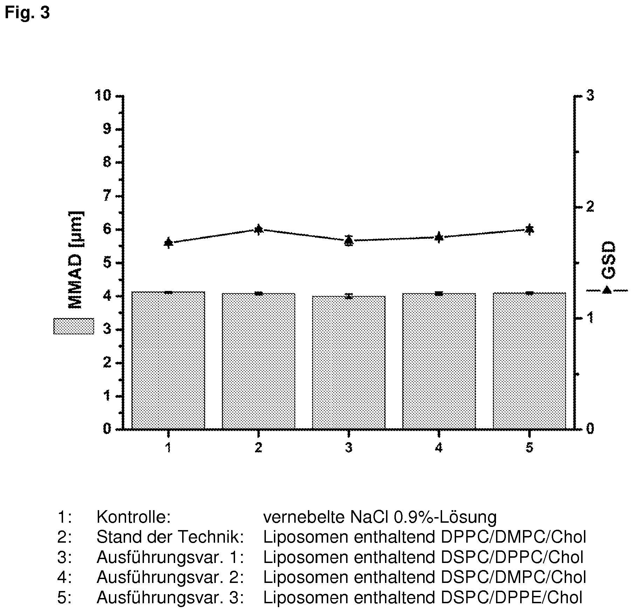

Auch die in Fig. 3 dargestellte Größe der Aerosolpartikel, welche für die Alveolargängigkeit des Inhalats ein wichtiger Faktor ist, wird nach Vernebelung der Liposomen-Dispersion mit Hilfe von Laser-Lichtstreuung bestimmt. Man erkennt, dass Partikel, welche die beispielhaft gezeigten erfindungsgemäßen Liposomen enthalten, einen medianen aerodynamischen Massendurchmesser MMAD von etwa 4 $\mu$m aufweisen und somit gut in die Alveolarräume der Lunge gelangen können.

In der Fig. 3 zeigt Säule 1 den medianen aerodynamischen Massendurchmesser und die geometrische Standardabweichung der Partikel einer als Kontrolle verwendeten und vernebelten 0,9%igen NaCl-Lösung, Säule 2 den medianen aerodynamischen Massendurchmesser und die geometrische Standardabweichung der Partikel einer aus dem Stand der Technik bekannten vernebelten liposomalen Formulierungen enthaltend DPPC/DMPC und CHOL, Säule 3 den medianen aerodynamischen Massendurchmesser und die geometrische Standardabweichung der Partikel einer vernebelten liposomalen Formulierung enthaltend DSPC/DPPC und CHOL entsprechend der Ausführungsvariante 1, Säule 4 den medianen aerodynamischen Massendurchmesser und die geometrische Standardabweichung der Partikel einer vernebelten liposomalen Formulierung enthaltend DSPC/DMPC und CHOL entsprechend der Ausführungsvariante 2 und Säule 5 den medianen aerodynamischen Massendurchmesser und die geometrische Standardabweichung der Partikel einer vernebelten liposomalen Formulierung enthaltend DSPC/DPPE und CHOL entsprechend der Ausführungsvariante 3.

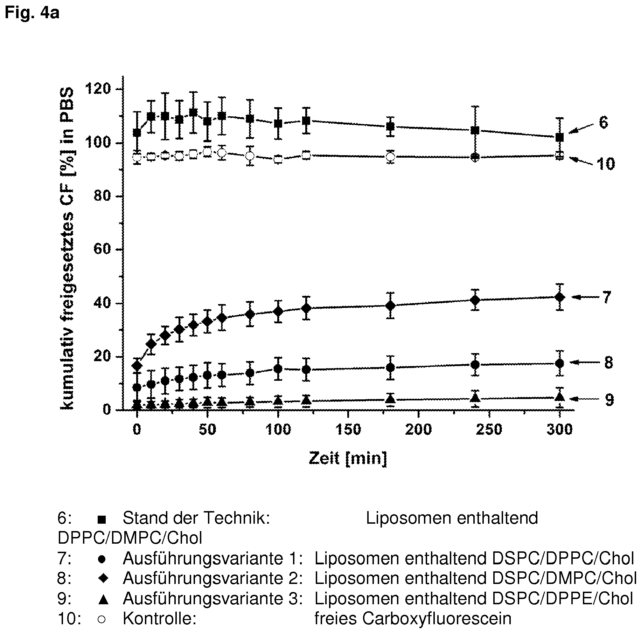

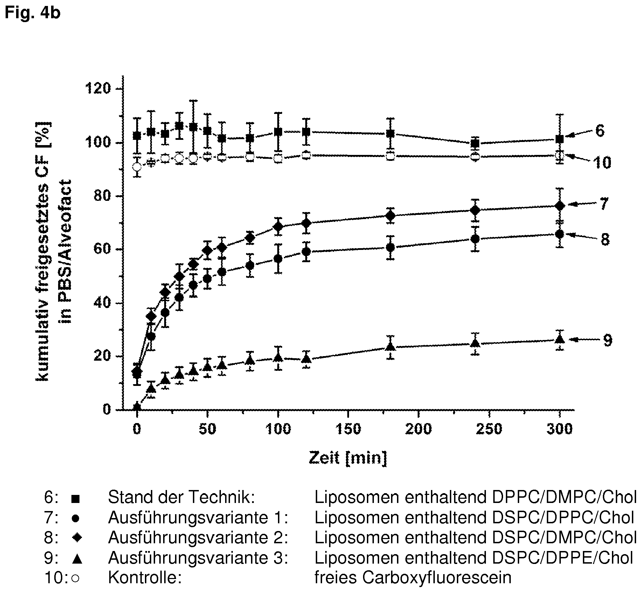

Zur Ermittlung der in den Figuren 4a und 4b dargestellten *in vitro*-Wirkstoff-Freisetzungs-Charakeristik der erfindungsgemäßen Liposomen werden jeweils 0,4 ml der wie oben beschrieben hergestellten Liposomen-Suspension in einem Freisetzungsmedium auf ein Volumen von 10 ml verdünnt. Als Freisetzungsmedium wird entweder PBS bei einem pH-Wert von 7.4 oder eine Lösung von 0,5 mg/ml Alveofact® in PBS verwendet. Die so hergestellte Dispersion wird bei 37°C inkubiert. In der ersten Stunde wird alle 10 Minuten eine Probe von 300 $\mu$l entnommen. In der zweiten Stunde wird alle 20 min eine Probe von 300 $\mu$l und in der dritten Stunde alle 30 min eine Probe von 300 $\mu$l entnommen. Die Proben werden jeweils sofort in einem Verhältnis von 1:20 mit 4°C kaltem PBS verdünnt und bis zur fluorimetrischen Bestimmung der Carboxyfluorescein-Freisetzung in lichtgeschützten Reaktionsgefäßen auf Eis gelagert.

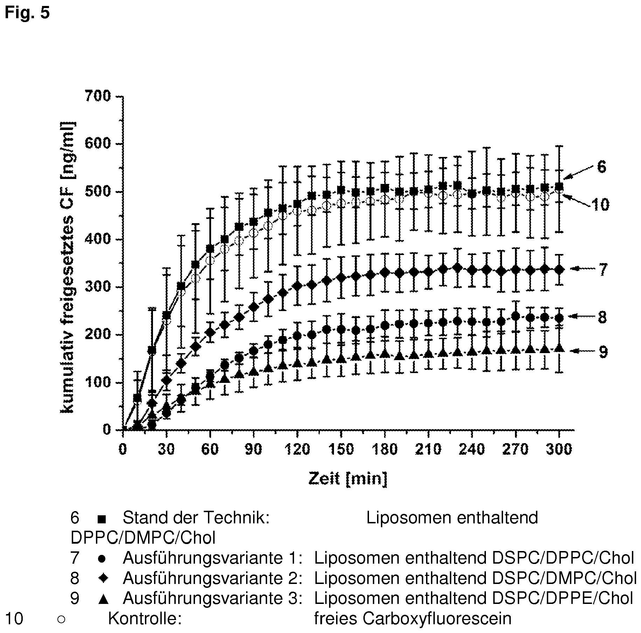

Zur Bestimmung der in Fig. 5 dargestellten Wirkstoff-Freisetzungs-Charakteristik wird als Modell die isolierte, perfundierte und ventilierte Kaninchenlunge verwendet (zur Beschreibung des an sich bekannten Modells siehe bspw. Lahnstein et al., International Journal of Pharmaceutics 351 (2008) Seiten 158 bis 164). Zur inhalativen Applikation werden wie oben beschrieben liposomale Formulierungen hergestellt, in PBS mit einem pH-Wert von 7,4 verdünnt und vernebelt. Die Aerosoldeposition im isolierten Organ wird quantitativ bestimmt und daraus die initial in der Lunge deponierte Wirk- oder Farbstoffmenge berechnet. Um festzustellen, wie viel Wirkstoff nach der inhalativen Applikation tatsächlich in der Lunge verbleibt, werden über einen Zeitraum von 300 Minuten Proben aus dem Perfusat entnommen und auf diese Weise die Menge Wirkstoff bestimmt, die im zeitlichen Verlauf aus der Lunge in das Perfusat übergetreten ist. Bei Kenntnis der ursprünglich in der Lunge deponierten Wirkstoff-Menge kann dann auf den Sustained release-Effekt der jeweiligen liposomalen Formulierung geschlossen werden. Dabei ergibt sich die jeweils in der Lunge befindliche Wirkstoffmenge aus der Differenz der initial deponierten und der in das Perfusat übergetretenen Wirkstoffmenge. Der Sustained Release-Effekt der inhalativ applizierten liposomalen Formulierungen ist umso stärker ausgeprägt, je weniger Wirkstoff pro Zeiteinheit aus der Lunge in das Perfusat übertritt.

In den Figuren 4a, 4b und 5 zeigen die Graphen 6 kumulativ aus Liposomen, enthaltend DPPC/DMPC/CHOL, freigesetztes CF, die Graphen 7 kumulativ aus Liposomen, enthaltend DSPC/DPPC/CHOL, freigesetztes CF entsprechend Ausführungsvariante 1, die Graphen 8 kumulativ aus Liposomen, enthaltend DSPC/DMPC/CHOL, freigesetztes CF entsprechend Ausführungsvariante 2, die Graphen 9 kumulativ aus Liposomen, enthaltend DSPC/DMPE/CHOL, freigesetztes CF entsprechend Ausführungsvariante 3 und die Graphen 10 kumulativ freigesetztes CF aus einer als Kontrolle verwendeten Lösung, enthaltend unverkapseltes CF.

Die Figuren 4a, 4b, und 5 lassen erkennen, dass Liposomen, die - wie eingangs beschrieben - aus dem Stand der Technik bekannt sind und aus einer Mischung von DPPC/DMPC und Cholesterin bestehen, den Modell-Wirkstoff Carboxyfluorescein CF unmittelbar und innerhalb kürzester Zeit komplett in Form einer Burst release-Freisetzung abgeben. Die Freisetzungskinetik ist dabei gut mit der von nicht-verkapseltem freien Carboxyfluorescein CF vergleichbar. So erkennt man in den *in vitro*-Charakteristiken der Figuren 4a und 4b, dass Liposomen, bestehend aus DPPC/DMPC und Cholesterin das enthaltene Carboxyfluorescein CF unmittelbar nach dem Start der Inkubation vollständig freisetzen. Dagegen ist in bei den erfindungsgemäßen Liposomen eine deutlich verlangsamte Freisetzung über einen längeren Zeitraum beobachtbar.

Auch im Organmodell zeigen die aus dem Stand der Technik bekannten Liposomen einen schnellen Anstieg der Carboxyfluorescein-Konzentration im Perfusat. Man erkennt gut, dass dieser vergleichbar mit dem Anstieg der Carboxyfluorescein-Konzentration im Perfusat nach inhalativer Applikation einer Lösung aus unverkapseltem Carboxyfluorescein CF ist. So wird bei beiden Formulierungen ein stabiles Plateau der CF-Perfustakonzentration nach ca. 140 Minuten von etwa 500 mg/ml erreicht. Die erfindungsgemäßen Liposomen (entsprechend beispielsweise einer der Ausführungsvarianten 1, 2 oder 3) zeigen dagegen auch im Organmodell einen deutlich langsameren Anstieg der Carboxyfluorescein-Konzentration im Perfusat mit jeweils einer deutlich geringeren CF-Konzentration am Versuchsende nach 300 Minuten. Aus diesen Daten erkennt man, dass im Falle der erfindungsgemäßen liposomalen Formulierungen, über einen längeren Zeitraum eine deutlich größere Menge an Carboxyfluorescein im Sinne eines Sustained release in der Lunge verbleibt.

Zusammenfassend erkennt man in den Figuren 4a, 4b, und 5 sehr deutlich und gut den Vorteil, den die erfindungsgemäßen Liposomen bieten, in dem sie über einen langen Zeitraum nur wenig Wirkstoff freisetzen und so eine Sustained release-Freisetzung ermöglichen. Weitere Vorteile zeigen sich bei Betrachtung der im Folgenden beispielhaft gezeigten Ausführungsvarianten.

**Ausführungsvariante 1**

Wie in Fig. 1 dargestellt, können die erfindungsgemäßen Liposomen beispielsweise als erstes Phospholipid Distearoylphosphatidylcholin DSPC, als zweites Phospholipid Dipalmitoylphosphatidylcholin DPPC und Cholesterin CHOL in einem molaren Verhältnis von DSPC:DPPC:CHOL = 4:4:2 enthalten. Das erste und das zweite Phospholipid liegen mithin im Verhältnis 1:1 vor, das Verhältnis von Phospholipiden zu Cholesterin beträgt 4:1.

Der Durchmesser solcher Liposomen beträgt nach der Extrusion $0,59 \pm 0,03~\mu m$, nach der Zentrifugation $0,59 \pm 0,04~\mu m$ und nach Vernebelung $0,59 \pm 0,02~\mu m$. Man erkennt, dass die Größe der erfindungsgemäßen Liposomen während der Vernebelung höchst stabil ist.

Die Wirkstoff-Ladungs-Effizienz beträgt, wie in Fig. 2 dargestellt, $1,29 \pm 0,18~\%$. Die ebenfalls der Fig. 2 entnehmbare Stabilität gegenüber Vernebelung ergibt sich aus dem Vergleich des Anteils vor der Vernebelung eingekapselten Modell-Wirkstoffs $CF_{lip}PRE$, der 96,1 % beträgt mit dem Anteil nach der Vernebelung eingekapselten Modell-Wirkstoffs $CF_{lip}POST$, der 79,1 % beträgt. Man erkennt, dass die erfindungsgemäßen Liposomen dieses Ausführungsbeispiels eine Vernebelungsstabilität von mehr als 80 % aufweisen, nämlich 82 %.

Aerosolpartikel, die Liposomen entsprechend dieses Ausführungsbeispiels enthalten, haben nach der Vernebelung mit einem Aeroneb® Professional-Vernebler einen medianen aerodynamischen Durchmesser (MMAD) von $4,08 \pm 0,04~\mu m$ bei einer geometrischen Standardabweichung (GSD) von 1,7, wie in Fig. 3 gezeigt. Man erkennt, dass die erfindungsgemäßen Liposomen vorteilhaft so vernebelbar sind, dass sie aufgrund der Größe der gebildeten Aerosolpartikel gut in den Alveolarraum der Lunge gelangen können.

Die errechnete Phasenübergangstemperatur der liposomalen Formulierung liegt bei 53°C, die gemessene, tatsächliche Phasenübergangstemperatur der Liposomen beträgt dagegen 46°C und mithin mehr als 37°C. Dies ist insbesondere im Hinblick auf eine verzögerte Wirkstofffreisetzung vorteilhaft. Die Liposomen bleiben dann nämlich bei Körpertemperatur entsprechend stabil und setzen den eingeschlossenen Wirkstoff nur langsam und in geringen Mengen frei. Dies zeigt sowohl die in den Figuren 4a und 4b dargestellte *in vitro* Wirkstoff-Freisetzungs-Charakteristik als auch die in der Fig. 5 dargestellte Wirkstoff-Freisetzungs-Charakteristik im Organmodell.

Bei einer Dispersion der erfindungsgemäßen Liposomen in PBS (vgl. Fig. 4a) wird *in vitro* während der ersten zwei Stunden eine ständig ansteigende Menge des Modell-Wirkstoffs freigesetzt, bis am Ende des Experiments nach 300 Minuten etwa 42% des Modell-Wirkstoffs freigesetzt sind. Werden die erfindungsgemäßen Liposomen in eine Dispersion mit PBS/Surfactant gebracht, so werden in den ersten zwei Stunden etwa 70 % des eingekapselten Modell-Wirkstoffes freigesetzt.

Bei der Bestimmung der Wirkstoff-Freisetzungscharakterstik im Organmodell (vgl. Fig. 5) zeigt die erfindungsgemäße Formulierung eine deutlich geringere Konzentration von Carboxyfluorescein im Perfusat, als es beispielsweise bei Formulierungen, die aus dem Stand der Technik bekannt sind, der Fall ist. Nach ca. 160 min Perfusion wird dabei ein Plateau erreicht, bei dem die Carboxyfluorescein-Konzentration im Perfusat ca. 320 ng/ml beträgt. Bis zum Ende der Beobachtung steigt die Konzentration dann nur noch geringfügig auf etwa 340 ng/ml. Man erkennt daran, dass die CF-Freisetzung in der Lunge aus dieser liposomalen-Formulierung verzögert erfolgt.

## Ausführungsvariante 2

Entsprechend einem weiteren Ausführungsbeispiel können die erfindungsgemäßen Liposomen auch als erstes Phospholipid Distearoylphosphatidylcholin (DSPC), als zweites Phospholipid Dimyristoylphosphatidylcholin (DMPC) und Cholesterin in einem molaren Verhältnis von DSPC:DMPC:CHOL = 6:1:2 enthalten. Das erste und das zweite Phospholipid liegen mithin im Verhältnis 6:1 vor, das Verhältnis von Phospholipiden zu Cholesterin beträgt 7:2 (= 3,5:1).

Der Durchmesser der Liposomen beträgt nach der Extrusion 0,60±0,02 μm, nach der Zentrifugation 0,61 ±0,02 μm und nach Vernebelung 0,64±0,09 μm. Man erkennt, dass die Größe der erfindungsgemäßen Liposomen während der Vernebelung höchst stabil ist.

Die Wirkstoff-Ladungs-Effizienz beträgt, wie in Fig. 2 dargestellt, 1,99 ± 0,21 %. Die ebenfalls der Fig. 2 entnehmbare Stabilität gegenüber Vernebelung ergibt sich aus dem Vergleich des Anteils vor der Vernebelung eingekapselten Modell-Wirkstoffs $CF_{lip}PRE$, der 96,6% beträgt mit dem Anteil nach der Vernebelung eingekapselten Modell-Wirkstoffs $CF_{lip}POST$, der 80,3% beträgt. Man erkennt, dass die erfindungsgemäßen Liposomen dieses Ausführungsbeispiels eine Vernebelungsstabilität von mehr als 80% aufweisen, nämlich 83 %.

Aerosolpartikel, die Liposomen entsprechend dieses Ausführungsbeispiels enthalten, haben nach der Vernebelung mit einem Aeroneb® Professional-Vernebler einen MMAD von 4,00 ± 0,06 μm bei einer GSD von 1,7, wie in Fig. 3 gezeigt. Man erkennt, dass die erfindungsgemäßen Liposomen vorteilhaft so vernebelbar sind, dass sie aufgrund der Größe der gebildeten Aerosolpartikel gut in den Alveolarraum der Lunge gelangen können.

Die errechnete Phasenübergangstemperatur der liposomalen Formulierung beträgt 56°C, und mithin mehr als 37°C. Dies ist insbesondere im Hinblick auf eine verzögerte Wirkstofffreisetzung vorteilhaft. Die Liposomen bleiben dann nämlich bei Körpertemperatur entsprechend stabil und setzen den eingeschlossenen Wirkstoff nur langsam und in geringen Mengen frei. Dies zeigt sowohl die in den Figuren 4a und 4b dargestellte *in vitro* Wirkstoff-Freisetzungs-Charakteristik als auch die in der Fig. 5 dargestellte Wirkstoff-Freisetzungs-Charakteristik im Organmodell.

Bei einer Dispersion der erfindungsgemäßen Liposomen in PBS (vgl. Fig. 4a) wird *in vitro* während der ersten zwei Stunden eine ständig ansteigende Menge des Modell-Wirkstoffs freigesetzt, bis am Ende des Experiments nach 300 Minuten etwa 18% des Modell-Wirkstoffs freigesetzt sind. Werden die erfindungsgemäßen Liposomen in eine Dispersion mit PBS/Surfactant gebracht, so werden in den ersten zwei Stunden etwa 60 % des eingekapselten Modell-Wirkstoffes freigesetzt.

Bei der Bestimmung der Wirkstoff-Freisetzungscharakterstik im Organmodell (vgl. Fig. 5) zeigt die erfindungsgemäße Formulierung eine deutlich geringere Konzentration von Carboxyfluorescein im Perfusat, als es beispielsweise bei Formulierungen, die aus dem Stand der Technik bekannt sind, der Fall ist. Nach ca. 160 min Perfusion wird dabei ein Plateau erreicht, bei dem die Carboxyfluorescein-Konzentration im Perfusat ca. 210 ng/ml beträgt. Bis zum Ende der Beobachtung steigt die Konzentration dann nur noch geringfügig auf etwa 240 ng/ml. Man erkennt daran, dass die CF-Freisetzung in der Lunge aus dieser liposomalen-Formulierung verzögert erfolgt.

## Ausführungsvariante 3

Wie in Fig. 1 dargestellt, enthalten die erfindungsgemäßen Liposomen als erstes Phospholipid Distearoylphosphatidylcholin DSPC, als zweites Phospholipid Dipalmitoylphosphatidylethanolamin DPPE und Cholesterin in einem molaren Verhältnis von DSPC:DPPE:CHOL = 6:2:2. Das erste und das zweite Phospholipid liegen mithin im Verhältnis 3:1 vor, das Verhältnis von Phospholipiden zu Cholesterin beträgt 4:1.

Der Durchmesser der Liposomen nach der Extrusion wurde mit 0,62 ± 0,02 μm, nach der Zentrifugation 0,62 ± 0,02 μm und nach Vernebelung 0,73 ± 0,13 μm. Man erkennt, dass die Größe der erfindungsgemäßen Liposomen während der Vernebelung höchst stabil ist.

Die Wirkstoff-Ladungs-Effizienz beträgt, wie in Fig. 2 dargestellt, 2,78 ± 0,30%. Die ebenfalls der Fig. 2 entnehmbare Stabilität gegenüber Vernebelung ergibt sich aus dem Vergleich des Anteils vor der Vernebelung eingekapselten Modell-Wirkstoffs $CF_{lip}PRE$, der 99,6 % beträgt mit dem Anteil nach der Vernebelung eingekapselten Modell-Wirkstoffs $CF_{lip}POST$, der 83,8 % beträgt. Man erkennt, dass die erfindungsgemäßen Liposomen dieses Ausführungsbeispiels eine Vernebelungsstabilität von mehr als 80 % aufweisen, nämlich 84 %.

Aerosolpartikel, die Liposomen entsprechend dieses Ausführungsbeispiels enthalten, haben nach der Vernebelung mit einem Aeroneb® Professional-Vernebler einen MMAD 4,09 ± 0,03 μm bei einer GSD von 1,8 wie in Fig. 3 gezeigt. Man erkennt, dass die erfindungsgemäßen Liposomen vorteilhaft so vernebelbar sind, dass sie aufgrund der Größe der gebildeten Aerosolpartikel gut in den Alveolarraum der Lunge gelangen können.

Die errechnete Phasenübergangstemperatur der liposomalen Formulierung liegt bei 60°C, die gemessene, tatsächliche Phasenübergangstemperatur der Liposomen beträgt dagegen etwa 55°C und mithin mehr als 37°C. Dies ist insbesondere im Hinblick auf eine verzögerte Wirkstofffreisetzung vorteilhaft. Die Liposomen bleiben dann nämlich bei Körpertemperatur entsprechend stabil und setzen den eingeschlossenen Wirkstoff nur langsam und in geringen Mengen frei. Dies zeigt sowohl die in den Figuren 4a und 4b dargestellte *in-vitro* Wirkstoff-Freisetzungs-Charakteristik als auch die in der Fig. 5 dargestellte Wirkstoff-Freisetzungs-Charakteristik im Organmodell.

Bei einer Dispersion der erfindungsgemäßen Liposomen in PBS (vgl. Fig. 4a) wurden nach 300 Minuten Beobachtungszeit etwa 5% des Modell-Wirkstoffes freigesetzt, bei einer Dispersion in PBS/Surfactant im gleichen Zeitraum etwa 26%.

Bei der Bestimmung der Wirkstoff-Freisetzungscharakterstik im Organmodell (vgl. Fig. 5) zeigt die erfindungsgemäße Formulierung eine deutlich geringere Konzentration von Carboxyfluorescein im Perfusat, als es beispielsweise bei Formulierungen, die aus dem Stand der Technik bekannt sind, der Fall ist. Nach ca. 110 min Perfusion steigt die Carboxyfluorescein-Konzentration im Perfusat auf ca. 130 ng/ml, danach erfolgt ein langsamerer Anstieg auf einen Endwert von 170 ng/ml nach 300 Minuten. Man erkennt daran, dass die CF-Freisetzung in der Lunge aus dieser liposomalen-Formulierung verzögert erfolgt.

Die Erfindung ist nicht auf eine der vorbeschriebenen Ausführungsformen beschränkt, sondern in vielfältiger Weise abwandelbar.

Sämtliche aus den Ansprüchen, der Beschreibung und den Zeichnungen hervorgehenden Merkmale und Vorteile, einschließlich konstruktiver Einzelheiten, räumlicher Anordnungen und Verfahrensschritten, können sowohl für sich als auch in den verschiedensten Kombinationen erfindungswesentlich sein.

Man erkennt, dass Liposomen für die pulmonale Applikation, vorteilhaft mindestens ein erstes und mindestens ein zweites Phospholipid, sowie Cholesterin und mindestens einen Wirk- und/oder Farbstoff enthalten, wobei das erste Phospholipid das Phosphatidylcholin Disteaorylphosphatidylcholin DSPC ist, und das zweite Phospholipid ein Phosphatidylcholin oder ein Ethanolamin ist, bevorzugt ausgewählt aus der Gruppe Dimyristoylphosphatidylcholin DMPC, Dipalmitoylphosphatidylcholin DPPC, Dipalmitoylphosphatidylethanolamin DPPE. Man erkennt weiter, dass das zweite Phospholipid bevorzugt Dimyristoylphosphatidylcholin DMPC oder Dipalmitoylphosphatidylethanolamin DPPE, besonders bevorzugt Dipalmitoylphosphatidylethanolamin DPPE ist. Dabei ist es günstig, wenn das erste und das zweite Phospholipid in einem molaren Verhältnis von 0,5:1 bis 10:1, bevorzugt in einem Verhältnis von 6:1 bis 2:1, besonders bevorzugt in einem Verhältnis von 3:1 vorliegen. Weiterhin ist es vorteilhaft, wenn das molare Verhältnis zwischen Phospholipiden und Cholesterin zwischen 10:1 und 1:1, bevorzugt zwischen 6:1 und 3:1, besonders bevorzugt 4:1 beträgt. Besonders günstig ist es außerdem, wenn die Liposomen eine Vernebelungsstabilität von mehr als 50%, bevorzugt mehr als 75%, besonders bevorzugt mehr als 80% aufweisen. Dabei beträgt die Größe der Liposomen zwischen 0,05 µm und 5 µm, bevorzugt zwischen 0,2 µm und 2,0 µm und der mediane aerodynamische Massendurchmesser von Aerosolpartikeln enthaltend die Liposomen beträgt zwischen 1 µm und 6 µm, bevorzugt zwischen 1,5 µm und 5 µm besonderes bevorzugt zwischen 2 µm und 4,5 µm. Man erkennt, dass es günstig ist, wenn sich die Größe der Liposomen nach der Vernebelung um weniger als 1 µm, bevorzugt weniger als 0,2 µm von der Größe der Liposomen vor der Vernebelung unterscheidet und wenn die Übergangstemperatur mehr als 37°C, bevorzugt mehr als 45°C, besonders bevorzugt mehr als 50°C beträgt. Man erkennt weiter, dass die Liposomen mit piezoelektrischen-, Düsen-, Ultraschall- Aerosolgeneratoren oder Soft-Mist-Inhalern vernebelbar sind.

Günstig ist es, wenn der Wirkstoff ausgewählt ist aus der Gruppe Appetitzügler/Antiadiposita, Azidosetherapeutika, Analeptika/Antihypoxämika, Analgetika, Antirheumatika, Anthelminthika, Antiallergika, Antianämika, Antiarrhythmika, Antibiotika, Antiinfektiva, Antidementiva, Antidiabetika, Antidota, Antiemetika, Antivertiginosa, Antiepileptika, Antihämorrhagika, Hämostatika, Antihypertonika, Antihypoglykämika, Antihypotonika, Antikoagulantia, Antiomykotika, Antiparasitika, Antiphlogistika, Antitussiva, Expektorantia, Arteriosklerosemittel, ß-Blocker, Kalzium-Kanal-Blocker, Hemmstoffe des Renin-Angiotensin-Aldosteron-Systems, Broncholytika, Antiasthmatika, Cholagoga, Gallenwegstherapeutika, Cholinergika, Kortikoide, Diagnostika und Mittel zur Diagnosevorbereitung, Diuretika, Durchblutungsfördernde Mittel, Entwöhnungsmittel, Enzyminhibitoren, Enzymaktivatoren oder -stimulatoren, Präparate bei Enzymmangel, Rezeptorantagonisten, Transportproteine, Fibrinolytika, Geriatrika, Gichtmittel, Grippemittel, Mittel gegen Erkältungskrankheiten, Gynäkologika, Hepatika, Hypnotika, Sedativa, Hypophysen- oder Hypothalamushormone, regulatorische Peptide, Hormone, Peptid-Hemmstoffe, Immunmodulatoren, Kardiaka, Koronarmittel, Laxantia, Lipidsenker, Lokalanästhetika, Neuraltherapeutika, Magen-Darm-Mittel, Migränemittel, Mineralstoffpräparate, Muskelrelaxanzien, Narkosemittel, Neurotrope Mittel, Osteoporosemittel, Kalzium-/ Knochenstoffwechsel-Regulatoren, Parkinsonmittel, Psychopharmaka, Sinusitismittel, Roborantia, Schilddrüsentherapeutika, Sera, Immunglobuline, Impfstoffe, Antikörper, Sexualhormone und ihre Hemmstoffe, Spasmolytika, Anticholinergika, Thrombozytenaggregationshemmer, Tuberkulosemittel, Urologika, Venentherapeutika, Vitamine, Zytostatika, Antineoplatische Mittel, Homöopathika, Gefäßaktive Substanzen, Gentherapeutika (DNA/RNA-Derivate), Transkriptions-Inhibitoren, Virostatika, Nikotin, Mittel gegen erektile Dysfunktion, Stickstoffmonoxid und/oder Stickstoffmonoxid-liberierende Substanzen. Vorteilhaft kann der Wirk- und/oder Farbstoff auch aus magnetischen Partikeln bestehen oder magnetische Partikel beinhalten.

Vorteilhaft ist außerdem eine Verwendung der erfindungsgemäßen Liposomen zur Herstellung eines pharmazeutischen Mittels zur Prävention, Diagnose und/oder Behandlung von Lungenerkrankungen und/oder systemischen Erkrankungen, sowie insbesondere die Verwendung solcher Liposomen zur Herstellung eines pharmazeutischen Mittels zur Prävention, Diagnose und/oder Behandlung von Erkrankungen des Alveolarraumes, die Verwendung solcher Liposomen zur Herstellung eines pharmazeutischen Mittels zur Prävention, Diagnose und/oder Behandlung von Erkrankungen der Atemwege und die Verwendung solcher Liposomen zur Herstellung eines pharmazeutischen Mittels zur Prävention, Diagnose und/oder Behandlung der pulmonalen Hypertonie.

**Abkürzungs- und Bezugszeichenliste**

| | |
|---|---|
| DSPC | Disteaorylphosphatidylcholin |
| DPPC | Dipalmitoylphosphatidylcholin |
| DMPC | Dimyristoylphosphatidylcholin |
| DPPE | Dipalmitoylphosethanolamin |

| | |
|---|---|
| P1 | erstes Phospholipid |
| P2 | zweites Phospholipid |
| PL | Phospholipide |

| | |
|---|---|
| Chol | Cholesterin |

| | |
|---|---|
| EE | Wirkstoff-Beladungs-Effizienz |
| CF | Carboxyfluorescein |
| CFstart | Wirkstoffmenge vor dem Entfernen nicht eingekapselten Wirkstoffs |
| CFencaps | Menge eingekapselten Wirkstoffs |
| CFtot | Gesamtkonzentration Wirkstoff |
| CFfree | Konzentration freien Wirkstoffs |
| CFlipPRE | Anteil vor der Vernebelung eingekapselten Wirkstoffs |
| CFlipPOST | Anteil nach der Vernebelung eingekapselten Wirkstoffs |

| | |
|---|---|
| MMAD | medianer aerodynamischer Massendurchmesser |
| MVD | medianer Volumen-Durchmesser |
| GSD | geometrische Standardabweichung |

| | |
|---|---|
| Vern. | Vernebelung |
| 1 | medianer aerodynamischer Massendurchmesser und geometrische Standardabweichung der Partikel einer vernebelten 0,9%igen NaCl-Lösung |
| 2 | medianer aerodynamischer Massendurchmesser und geometrische Standardabweichung der Partikel einer vernebelten liposomalen Formulierungen enthaltend DPPC/DMPC und CHOL |
| 3 | medianer aerodynamischer Massendurchmesser und geometrische Standardabweichung der Partikel einer vernebelten liposomalen Formulierung enthaltend DSPC/DPPC und CHOL |
| 4 | medianer aerodynamischer Massendurchmesser und geometrische Standardabweichung der Partikel einer vernebelten liposomalen Formulierung enthaltend DSPC/DMPC und CHOL |
| 5 | medianer aerodynamischer Massendurchmesser und geometrische Standardabweichung der Partikel einer vernebelten liposomalen Formulierung enthaltend DSPC/DPPE und CHOL |
| 6 | kumulativ aus Liposomen enthaltend DPPC/DMPC/CHOL freigesetztes CF |
| 7 | kumulativ aus Liposomen enthaltend DSPC/DPPC/CHOL freigesetztes CF |
| 8 | kumulativ aus Liposomen enthaltend DSPC/DMPC/CHOL freigesetztes CF |
| 9 | kumulativ aus Liposomen enthaltend DSPC/DMPE/CHOL freigesetztes CF |
| 10 | kumulativ freigesetztes CF aus einer Lösung enthaltend unverkapseltes CF |

**Patentansprüche**

1. Liposomale Formulierung für die pulmonale Applikation mittels Vernebelung, wobei die Formulierung enthält:

   A) Liposomen umfassend

   i) ein erstes Phospholipid, das Disteaorylphosphatidylcholin (DSPC) ist,
   ii) ein zweites Phospholipid, das ein Phosphatidylcholin oder ein Ethanolamin ist, ausgewählt aus der Gruppe bestehend aus Dimyristoylphosphatidylcholin (DMPC), Dipalmitoylphosphatidylcholin (DPPC) und Dipalmitoylphosphatidylethanolamin (DPPE), und
   iii) Cholesterin und

   B) mindestens einen in Liposomen eingeschlossenen Wirkstoff und/oder Farbstoff.

2. Liposomale Formulierung entsprechend Anspruch 1, **dadurch gekennzeichnet, dass** das erste und das zweite Phospholipid in einem molaren Verhältnis von 0,5:1 bis 10:1, bevorzugt in einem Verhältnis von 6:1 bis 2:1, besonders bevorzugt in einem Verhältnis von 3:1 vorliegen.

3. Liposomale Formulierung entsprechend einem der Ansprüche 1 bis 2, **dadurch gekennzeichnet, dass** das molare Verhältnis zwischen a) dem ersten und zweiten Phospholipid und b) Cholesterin zwischen 10:1 und 1:1, bevorzugt zwischen 6:1 und 3:1, besonders bevorzugt 4:1 beträgt.

4. Liposomale Formulierung entsprechend einem der Ansprüche 1 bis 3, **dadurch gekennzeichnet, dass** die Liposomen eine Vernebelungsstabilität von mehr als 50%, bevorzugt von mehr als 75%, besonders bevorzugt von mehr als 80% aufweisen.

5. Liposomale Formulierung entsprechend einem der Ansprüche 1 bis 4, **dadurch gekennzeichnet, dass** die Größe der Liposomen zwischen 0,05 $\mu$m und 5 $\mu$m, bevorzugt zwischen 0,2 $\mu$m und 2,0 $\mu$m beträgt.

6. Liposomale Formulierung entsprechend einem der Ansprüche 1 bis 5, **dadurch gekennzeichnet, dass** der mediane aerodynamische Massendurchmesser (MMAD) von Aerosolpartikeln enthaltend die Liposomen zwischen 1 $\mu$m und 6 $\mu$m, bevorzugt zwischen 1,5 $\mu$m und 5 $\mu$m besonderes bevorzugt zwischen 2 $\mu$m und 4,5 $\mu$m beträgt.

7. Liposomale Formulierung entsprechend einem der Ansprüche 1 bis 6, **dadurch gekennzeichnet, dass** sich die Größe der Liposomen nach der Vernebelung um weniger als 1 $\mu$m, bevorzugt weniger als 0,2 $\mu$m von der Größe der Liposomen vor der Vernebelung unterscheidet.

8. Liposomale Formulierung entsprechend einem der Ansprüche 1 bis 7, **dadurch gekennzeichnet, dass** die Phasenübergangstemperatur mehr als 37°, bevorzugt mehr als 45°, besonders bevorzugt mehr als 50°C beträgt.

9. Liposomale Formulierung entsprechend einem der Ansprüche 1 bis 8, **dadurch gekennzeichnet, dass** die Liposomen mit Piezoeleketrischen-, Düsen-, Ultraschall-Aerosolgeneratoren oder Soft-Mist-Inhalern vernebelbar sind.

10. Liposomale Formulierung entsprechend Anspruch 1, **dadurch gekennzeichnet, dass** der Wirkstoff bildgebend und/oder radioaktiv und/oder ein Kontrastmittel ist.

11. Liposomale Formulierung entsprechend einem der Ansprüche 1 bis 9, **dadurch gekennzeichnet, dass** der Wirk- und/oder Farbstoff aus magnetischen Partikeln besteht oder magnetische Partikel beinhaltet.

12. Liposomale Formulierung entsprechend einem der Ansprüche 1 bis 11 zur Verwendung bei der Prävention, Diagnose und/oder Behandlung von Lungenerkrankungen, von systemischen Erkrankungen, von Erkrankungen des Alveolarraumes oder von Erkrankungen der Atemwege.

13. Liposomale Formulierung entsprechend einem der Ansprüche 1 bis 11 zur Verwendung bei der Prävention, Diagnose und/oder Behandlung der pulmonalen Hypertonie.

14. Liposomale Formulierung entsprechend einem der Ansprüche 1 bis 11 und liposomale Formulierung zur Verwendung bei der Prävention, Diagnose und/oder Behandlung entsprechend einem der Ansprüche 12 bis 13, **dadurch gekennzeichnet, dass** der Wirkstoff Treprostinil, Iloprost oder Sildenafil enthält.

**Claims**

1. Liposomal formulation for pulmonary administration by nebulisation, wherein the formulation contains:

   A) liposomes comprising:

   i) a first phospholipid, which is disteaorylphosphatidylcholine (DSPC);
   ii) a second phospholipid which is a phosphatidylcholine or an ethanolamine, selected from the group consisting of dimyristoylphosphatidylcholine (DMPC), dipalmitoylphosphatidylcholine (DPPC), and dipalmitoylphosphatidylethanolamine (DPPE), and
   iii) cholesterol; and

   B) at least one active agent and/or dye encapsulated in liposomes.

2. Liposomal formulation according to claim 1, **characterised in that** the first and the second phospholipid are in a molar ratio of 0.5:1 to 10:1, preferably in a ratio of 6:1 to 2:1, particularly preferably in a ratio of 3:1.

3. Liposomal formulation according to any of claims 1 to 2, **characterised in that** the molar ratio between a) the first and the second phospholipid and b) the cholesterol is between 10:1 and 1:1, preferably between 6:1 and 3:1, particularly preferably 4:1.

4. Liposomal formulation according to any of claims 1 to 3, **characterised in that** the liposomes have a nebulisation stability of more than 50%, preferably of more than 75%, particularly preferably of more than 80%.

5. Liposomal formulation according to any of claims 1 to 4, **characterised in that** the size of the liposomes is between 0.05 $\mu$m and 5 $\mu$m, preferably between 0.2 $\mu$m and 2.0 $\mu$m.

**6.** Liposomal formulation according to any of claims 1 to 5, **characterised in that** the mass median aerodynamic diameter (MMAD) of aerosol particles containing the liposomes is between 1 μm and 6 μm, preferably between 1.5 μm and 5 μm, particularly preferably between 2 μm and 4.5 μm.

**7.** Liposomal formulation according to any of claims 1 to 6, **characterised in that** the size of the liposomes after nebulisation differs by less than 1 μm, preferably less than 0.2 μm from the size of the liposomes before nebulisation.

**8.** Liposomal formulation according to any of claims 1 to 7, **characterised in that** the phase transition temperature is higher than 37°C, preferably higher than 45°C, particularly preferably higher than 50°C.

**9.** Liposomal formulation according to any of claims 1 to 8, **characterised in that** the liposomes can be nebulised by piezoelectric, air-jet, ultrasonic aerosol generators or soft-mist inhalers.

**10.** Liposomal formulation according to claim 1, **characterised in that** the active agent is image-producing and/or radioactive and/or a contrast agent.

**11.** Liposomal formulation according to any of claims 1 to 9, **characterised in that** the active agent and/or dye consists of magnetic particles or contains magnetic particles.

**12.** Liposomal formulation according to any of claims 1 to 11 for use in the prevention, diagnosis and/or treatment of lung diseases, of systemic diseases, of diseases of the alveolar cavity or diseases of the respiratory tract.

**13.** Liposomal formulation according to any of claims 1 to 11 for use in the prevention, diagnosis and/or treatment of pulmonary hypertony.

**14.** Liposomal formulation according to any of claims 1 to 11 and liposomal formulation for use in the prevention, diagnosis and/or treatment according to any of claims 12 to 13, **characterised in that** the active agent contains treprostinil, iloprost or sildenafil.

**Revendications**

**1.** Formulation liposomale destinée à une application pulmonaire par nébulisation, dans laquelle la formulation contient :

A) des liposomes comprenant

i) un premier phospholipide qui est la distéorylphosphatidylcholine (DSPC),
ii) un second phospholipide, qui est une phosphatidylcholine ou une éthanolamine, choisi dans le groupe constitué par la dimyristoylphosphatidylcholine (DMPC), la dipalmitoylphosphatidylcholine (DPPC) et la dipalmitoylphosphatidyléthanolamine (DPPE), et
iii) du cholestérol et

B) au moins un principe actif et/ou colorant enfermé dans des liposomes.

**2.** Formulation liposomale selon la revendication 1, **caractérisée en ce que** le premier et le second phospholipide sont à un rapport molaire de 0,5:1 à 10:1, de préférence de 6:1 à 2:1, le plus préférentiellement à un rapport de 3:1.

**3.** Formulation liposomale selon l'une des revendications 1 à 2, **caractérisée en ce que** le rapport molaire entre a) le premier et le second phospholipide et b) le cholestérol est compris entre 10:1 et 1:1, de préférence entre 6:1 et 3:1, le plus préférentiellement est de 4:1.

**4.** Formulation liposomale selon l'une des revendications 1 à 3, **caractérisée en ce que** les liposomes présentent une stabilité à la nébulisation supérieure à 50 %, de préférence supérieure à 75 %, le plus préférentiellement supérieure à 80 %.

**5.** Formulation liposomale selon l'une des revendications 1 à 4, **caractérisée en ce que** la taille des liposomes est comprise entre 0,05 μm et 5 μm, de préférence entre 0,2 μm et 2,0 μm.

**6.** Formulation liposomale selon l'une des revendications 1 à 5, **caractérisée en ce que** le diamètre aérodynamique massique médian (MMAD) de particules d'aérosols contenant les liposomes est compris entre 1 μm et 6 μm, de préférence entre 1,5 μm et 5 μm, le plus préférentiellement entre 2 μm et 4,5 μm.

**7.** Formulation liposomale selon l'une des revendications 1 à 6, **caractérisée en ce que** la taille des liposomes après nébulisation diffère de moins de 1 μm, de préférence de moins de 0,2 μm, de la taille des liposomes avant nébulisation.

**8.** Formulation liposomale selon l'une des revendications 1 à 7, **caractérisée en ce que** la température de transition de phase est supérieure à 37°, de préférence supérieure à 45°, le plus préférentiellement supérieure à 50°C.

**9.** Formulation liposomale selon l'une des revendications 1 à 8, **caractérisée en ce que** les liposomes peuvent être nébulisés avec des générateurs d'aérosols piézoélectriques, à buse, à ultrasons ou des inhalateurs à brouillard doux.

10. Formulation liposomale selon la revendication 1, **caractérisée en ce que** le principe actif est imageur et/ou radioactif et/ou un agent de contraste.

11. Formulation liposomale selon l'une des revendications 1 à 9, **caractérisée en ce que** le principe actif et/ou colorant est constitué de particules magnétiques ou contient des particules magnétiques.

12. Formulation liposomale selon l'une quelconque des revendications 1 à 11 pour une utilisation dans la prévention, le diagnostic et/ou le traitement des maladies pulmonaires, des maladies systémiques, des maladies de la cavité alvéolaire ou des maladies des voies respiratoires.

13. Formulation liposomale selon l'une quelconque des revendications 1 à 11 pour une utilisation de prévention, de diagnostic et/ou de traitement de l'hypertension pulmonaire.

14. Formulation liposomale selon l'une quelconque des revendications 1 à 11 et formulation liposomale pour une utilisation de prévention, de diagnostic et/ou de traitement selon l'une quelconque des revendications 12 à 13, **caractérisée en ce que** le principe actif contient du tréprostinil, de l'iloprost ou du sildénafil.

**Fig. 1**

| Ausführung s- variante | P1 | P2 | Verhältnis P1:P2: Chol | Verhältnis P1:P2 | Verhältnis PL:Chol |
|---|---|---|---|---|---|
| 1 | DSPC | DPPC | 4:4:2 | 1:1 | 4:1 |
| 2 | DSPC | DMPC | 6:1:2 | 6:1 | 3,5:1 |
| 3 | DSPC | DPPE | 6:2:2 | 3:1 | 4:1 |

**Fig. 2**

| | Ausführungsvar. 1 DSPC/DPPC/Chol | Ausführungsvar. 2 DSPC/DMPC/Chol | Ausführungsvar. 3 DSPC/DPPE/Chol |
|---|---|---|---|
| EE [%] | 1,29±0,18 | 1,99±0,21 | 2,78±0,30 |
| $CF_{lip}PRE$ [%] | 96,1±2,1 | 96,6±0,8 | 99,6±0,2 |
| $CF_{lip}POST$ [%] | 79,1±2,6 | 80,3±0,9 | 83,8±2,8 |
| Vernebelungs- stabilität | 82% | 83% | 84% |
| Liposomengröße vor Vern. [µm] | 0,59±0,04 | 0,61±0,02 | 0,62±0,02 |
| Liposomengröße nach Vern. [µm] | 0,59±0,02 | 0,64±0,09 | 0,73±0,13 |

**Fig. 3**

| 1: | Kontrolle: | vernebelte NaCl 0.9%-Lösung |
|---|---|---|
| 2: | Stand der Technik: | Liposomen enthaltend DPPC/DMPC/Chol |
| 3: | Ausführungsvar. 1: | Liposomen enthaltend DSPC/DPPC/Chol |
| 4: | Ausführungsvar. 2: | Liposomen enthaltend DSPC/DMPC/Chol |
| 5: | Ausführungsvar. 3: | Liposomen enthaltend DSPC/DPPE/Chol |

**Fig. 4a**

6:  ■ Stand der Technik:        Liposomen enthaltend DPPC/DMPC/Chol
7:  ● Ausführungsvariante 1: Liposomen enthaltend DSPC/DPPC/Chol
8:  ◆ Ausführungsvariante 2: Liposomen enthaltend DSPC/DMPC/Chol
9:  ▲ Ausführungsvariante 3: Liposomen enthaltend DSPC/DPPE/Chol
10: ○ Kontrolle:              freies Carboxyfluorescein

**Fig. 4b**

| 6: ■ | Stand der Technik: | Liposomen enthaltend DPPC/DMPC/Chol |
| 7: ● | Ausführungsvariante 1: | Liposomen enthaltend DSPC/DPPC/Chol |
| 8: ◆ | Ausführungsvariante 2: | Liposomen enthaltend DSPC/DMPC/Chol |
| 9: ▲ | Ausführungsvariante 3: | Liposomen enthaltend DSPC/DPPE/Chol |
| 10:○ | Kontrolle: | freies Carboxyfluorescein |

**Fig. 5**

6 ■ Stand der Technik:       Liposomen enthaltend DPPC/DMPC/Chol
7 ● Ausführungsvariante 1: Liposomen enthaltend DSPC/DPPC/Chol
8 ◆ Ausführungsvariante 2: Liposomen enthaltend DSPC/DMPC/Chol
9 ▲ Ausführungsvariante 3: Liposomen enthaltend DSPC/DPPE/Chol
10 ○ Kontrolle:             freies Carboxyfluorescein

**IN DER BESCHREIBUNG AUFGEFÜHRTE DOKUMENTE**

**In der Beschreibung aufgeführte Patentdokumente**

- DE 10214983 A
- US 2003232019 A1
- WO 8606959 A1
- WO 2004112695 A2
- US 2006141047 A1
- WO 03084507 A2

**In der Beschreibung aufgeführte Nicht-Patentliteratur**

- **KLEEMANN et al.** *Pharmaceutical Research,* Februar 2007, vol. 24 (2
- **LAHNSTEIN et al.** *International Journal of Pharmaceutics,* 2008, vol. 351, 158-164